# EUROPEAN PATENT SPECIFICATION

(11) **EP 0 955 347 B1**
(45) Date of publication and mention of the grant of the patent: **31.08.2011**
(21) Application number: 99303539.3
(22) Date of filing: 06.05.1999
(51) Int. Cl.: C09J 7/02, A61L 27/00

(54) **Method for adhering substrates using adhesive devices containing silicone gels**
Verfahren zum Kleben von Substraten mit Silikongel enthaltenden klebenden Vorrichtungen
Procédé de collage de substrats utilisant des dispositifs adhésifs contenant un gel de silicone

(30) Priority: 06.05.1998 EP 98480031
(43) Date of publication of application: 10.11.1999
(73) Proprietor: Dow Corning France S.A., 69432 Lyon Cedex 3 (FR)
(72) Inventor: Colas, Andre Rudolf Louis, 06560 Valbonne (FR); Lord, Gary, 06330 Roquefort les Pins (FR); Valencia, Marie Therese, 06580 Pegomas (FR); Thomas, Xavier, 06600 Antibes (FR)
(74) Representative: Gillard, Richard Edward

(56) References cited:
- EP-A- 0 308 216
- WO-A-86/00532
- WO-A-94/24964
- CA-A- 2 101 509
- DATABASE WPI Section Ch, Week 199825 Derwent Publications Ltd., London, GB; Class A26, AN 1998-279820 XP002236875 -& JP 10 095072 A (FUJIKURA RUBBER WORKS LTD), 14 April 1998 (1998-04-14)

## Description

The present invention relates to the use of a double sided adhesive device comprising silicone gels for adhering a medical prosthesis to the skin of human or animal body.

Silicone pressure sensitive adhesives (PSAs) and tapes containing such adhesives are known in the art and many are commercially available. Typically, silicone PSAs comprise condensed blends of silicone fluids and silicone resins. When used as tapes, typically such silicone PSAs are applied as thin coatings (e.g., < 0.1 mm) on one or both sides of a carrier material.

It is likewise known in the art to use silicone PSAs in medical applications. For instance, it is known to use silicone PSAs to adhere transdermal drug delivery devices and medical prosthesis to patients.

Silicone PSAs, however, can have a number of properties which limit their use in medical applications. For instance, the adhesive strength of silicone PSAs is often so great that a patient's skin or the object to be adhered can be damaged on removal of the PSA. Additionally, silicone PSAs often exhibit cold flow properties at skin temperature. As such, the resultant inflexible layers of PSA can be very uncomfortable on the patient's body. Finally, silicone PSAs often delaminate from the carrier leaving a coating of the PSA on the skin and/or the object to be adhered. Not only is this a cosmetic problem, but it also limits the ability to reuse the adhesive.

Silicone gels are also known in the art and described, for instance, in WO95/22997, WO96/09076 and EP300,620. These gels have been used, for example, as dielectrics, vibration dampers and in medical therapy for cutaneous scars or injuries (e.g., abrasions, surgical areas or burns). In this latter use, the silicone gel is in the form of a sheet with one tacky surface for adherence to the patient's skin and one non-tacky surface to inhibit undesirable adhesion to the gel (e.g., the patient's clothing)Particular reference is made to WO,96/09076, which discloses a hypertrophic scar dressing comprising a flexible carrier sheet having a silicone-gel forming continuous layers on both sides of the carrier material. The silicone-gel is tacky and skin-adherent on the side of the dressing which lies against the users skin when worn. The dressing has a thickness of 0.2-1.5 mm.

We have now discovered an adhesive device comprising silicone gels which can be used to provide a comfortable and convenient method for adhering a medical prosthesis to the skin of a human or animal body.

In addition, reference is made to the following prior art documents.

WO 94/24964 discloses a breast prosthesis which is connected near its edge to a holding strip which has a permanently adhesive layer for attachment to the breast of the bearer and, to form a longer-lasting attachment, the connection is situated only in the central area of the holding strip. CA 2 101 509 discloses an external breast enhancement to be attached to the chest employing a medical adhesive such as Dow Coming Corporation's Silastic®.

JP 10-95072 discloses a double-sided silicone coated tape having non-slip and sealing properties. The tape has silicone resin or rubber layers formed on both faces of a base material film.

WO 86/00532 discloses a silicone-based pressure sensitive adhesive for use with transdermal therapeutic devices. EP 0 308 216 discloses a double-sided adhesive tape with a low surface energy adhesive on at least one side, where the adhesive has superior properties when the adherend is of the low surface energy type and the surface energy of the adhesive is lower than that of the adherend.

Accordingly, in one of its aspects the present invention provides a method for adhering a first substrate to a second substrate with an adhesive device, the improvement comprising the use of an adhesive device comprising:
a carrier sheet, said carrier sheet having at least two surfaces;
on one surface of the carrier sheet is a first, continuous layer of a silicone gel having a density in the range of about 100 to 4500 g/m²; said gel having sufficient tack to adhere to the first substrate; and
on a second surface of the carrier sheet is a second continuous layer of a silicone gel having a density in the range of about 100 to 4500 g/m², said gel having sufficient tack to adhere to the second substrate,
wherein the first substrate is a prosthesis and the second substrate is the skin of a human or an animal body

The adhesion and physical properties of the adhesive devices used in the present invention can be tailored to specific end uses by modifying the gels.

In one preferred embodiment of the invention, one layer of gel in the adhesive device used in the present invention is sufficiently tacky to adhere to the skin of a human or animal body during its intended use and yet has sufficiently low tack to allow removal without discomfort. The second layer of gel in the adhesive device is sufficiently tacky to adhere to a medical prosthesis during its intended use and yet has sufficiently low tack to allow removal without damage. Because of this ease in removability and because the gel generally maintains its tack after removal, the devices of the invention can be reused. In addition, the reusability allows for easy and comfortable repositioning of medical prosthesis. Finally, silicone gels lack cold flow and, as such, are sufficiently soft to allow comfortable use by the human or animal.

In its most generic form, the adhesive device used in the present invention comprises a carrier sheet having continuous layers of silicone gel on two surfaces. The gel layers are designed to adhere to medical prosthesis and the skin of human or animal bodies, hereafter 'animal bodies' or 'patients'.

The carrier sheet of the adhesive device used in the present invention serves as support and reinforcement for the silicone gel. Such carrier sheets generally comprise thin, coherent materials. They can be continuous or they can have perforations such as holes, gaps, or spaces therein. Preferred materials comprise non-woven materials.

Suitable materials to be used as the carrier sheets are known in the art for other purposes and are commercially available. Representative examples include soft plastic films such as polyethylene, polyamide, polyurethane, nylon, polyester, polypropylene, polytetrafluoroethylene, silicones and the like and non-woven polysaccharide based materials.

The carrier sheets should be chosen for the desired application of the adhesive device. For adhering prosthesis to patients, the carrier sheet is generally sufficiently thin and pliable to allow for comfortable wear by the patient while retaining sufficient strength to insure integrity of the gel during use. Generally, the carrier sheet has a density of about 5 to 150 g/m² which generally corresponds to a thickness in the range of about 0.01 to about 1 mm. Preferably, the carrier sheet has a density of about 10 to 5og/m².

The silicone gel layers of the adhesive device used in the present invention should be chosen for the desired application of the adhesive device. If desired, different silicone gels can be used on each side of the carrier sheet such that the adhesion of the gels may vary.

For adhering prosthesis to patients, the gels should have sufficient tack to adhere to the body of the patient and to the prosthesis. The silicone gel should also be soft so that it is comfortable for the user and non-friable so that it is durable for its intended use.

The gels of the adhesive device used in the present invention are generally formed from linear or branched silicones having reactive groups thereon, as is known in the art. Such reactive groups undergo a crosslinking reaction during curing. Examples of crosslinking reactions include the hydrosilylation reaction in which a silicone having an Si-H reactive group reacts with a silicone having an aliphatically unsaturated reactive group in the presence of a platinum or rhodium catalyst. Alternatively, the reaction can involve the reaction of a silicone having an Si-OH reactive group with a silicone or a chain extender (e.g., a silane) having an alkoxy reactive group in the presence of a metal catalyst. In yet another alternative embodiment, a silicone having an Si-OH containing polymer is mixed with an alkoxysilane in the presence of a titanate catalyst. Other known cure mechanisms are also effective herein.

The preferred gels herein are obtained by reacting an alkenyl-substituted polydiorganosiloxane, preferably a polydimethylsiloxane having silicon-bonded vinyl, allyl or hexenyl groups, an organosiloxane containing silicon-bonded hydrogen atoms and a catalyst for the reaction of the SiH groups with the Si-alkenyl groups, such as a platinum metal or compounds or complexes thereof. Such compositions cure at normal ambient temperatures, but curing can be expedited by exposure to elevated temperatures, e.g., from about 40°C to about 120°C.

Preferred Si-H and Si-alkenyl siloxanes to be used in the above reaction have viscosities in the range of 5 to 60,000 mm2/second. The preferred ratio of (H as SiH)/(Alkenyl as Si-Alkenyl) is generally in the range of 0.1 to 10:1.

If desired, other components can be included in the gels of the present invention including, but not limited to, fillers, pigments, low temperature cure inhibitors, additives for improving adhesion, pharmaceutical agents, cosmetic agents, resins, fluids or other materials conventionally used in gels.

Suitable gels and gel forming compositions are described in, for example, G.B. Patents 849,885, 945,580 and 2,192,142, U.S. Patent 3,020,260, and EP261,167, EP300,620 and EP322,118.

The consistency, strength and tackiness of the gel is determined by a number of factors including the ratio of reactive groups in the materials, the viscosity of the polymers, and the like. One skilled in the art would know how to adjust this ratio to obtain a product with the properties desired for a given use.

As measured by the Cone Penetration Test method based on ASTM D-217-88, preferred gels have a penetration of 50 to 300 mm with a cone category 1806-1 weighted 62.5g.

The gels have a density in the range of about 100 to 4500 g/m² with densities in the range of about 150 to 1200 g/m² being preferred. Such gels would generally have thicknesses in the range of about 0.2 to about 5 mm with gels of 0.2 to 1.5 mm being preferred.

The adhesive strength of the silicone gels should be sufficient to maintain adhesion for the desired use. As the adhesive device is used to adhere a prosthesis to a patient, the adhesive strength of the gel used against the patient's body should be sufficient to ensure that the prosthesis remains attached to the patient and yet not so strong that excessive numbers of skin cells are removed when the adhesive device is removed. When measured with a Probe Tack Tester, the tack is generally between 50 and 500 g, with tack in the range of 150 to 350 g being preferred. The adhesive strength of the silicone gel used against the prosthesis is not as critical as that for the other gel layer. It should, however, be sufficient to ensure that adhesion is maintained without causing damage to the prosthesis when removed. As mentioned above, this can be the same or a different gel than the gel used for adherence to the patient body. Adhesive strengths are generally in the same range as that for the gel used for adherence to the patient body.

Since it is desired to have the gel adhere more strongly to the carrier sheet than either the patient's body or the prosthesis, an additional adhesive or an adhesion promoter may be used, if necessary, to bond the gel to the carrier sheet.

The silicone gels should be sufficiently soft and flexible to ensure comfort to the user. However, since softness also generally results in weaker gels, these two factors should be considered in selection and formulation of the gel.

If desired, the surfaces of the gels to be adhered to the patient and the prosthesis can be covered or protected with a release liner prior to use. Suitable release liner materials are known in the art and can include, for instance, a plastic or multi-ply material such as a silicone, a fluorinated silicone, a fluorine polymer, polyethylene, a PVC or the like. Additionally, the release liner could be made from a wide variety of materials (e.g., paper) coated with a suitable release coating. Finally, the surface of the release coating can be smooth, embossed or in any other desirable form.

The adhesive device used in the present invention can be made by any desirable technique. One example comprises preforming the gel (e.g., as a sheet) and the carrier sheet by known procedures e.g. by molding, calendering or casting and then bringing them together. For example, the gel may be preformed (e.g., as a sheet) by casting and curing the gel-forming composition on a suitable substrate. The carrier sheet may be preformed by calendering and then the carrier sheet applied over the gel. The second layer of gel can then be applied over the other exposed surface of the carrier sheet. Alternatively, the procedure may be reversed and the gel applied over the carrier sheet followed by applying the second gel layer over the exposed surface of the carrier sheet. If necessary, an adhesive may be employed to hold the components together in the laminated configuration.

Another method of making the adhesive device of this invention comprises (1) applying a gel forming composition to a substrate, (2) curing the gel forming composition to form the gel, (3) applying a carrier sheet precursor composition on the exposed surface of the gel, (4) curing the carrier sheet precursor to form the carrier sheet, (5) applying a second gel forming composition on the exposed surface of the carrier sheet and (6) curing the second gel forming composition.

Yet another suitable method comprises (1) applying a carrier sheet precursor composition to a substrate, (2) curing the carrier sheet precursor to form the carrier sheet, (3) applying a gel forming composition on one or both surfaces of the carrier sheet, and (4) curing the gel forming composition to form the gel(s). Obviously, if the gel was only formed on one surface of the carrier sheet during previous steps, the second layer of gel must be formed by a similar process.

In the above processes, the carrier sheet precursor composition and the gel forming composition may be applied by techniques such as dipping, spraying, coating, bar coating, etc. If desired, the carrier sheet precursor composition and the gel forming composition can be used as a dispersion or solution in a volatile solvent such as a organic solvent, a low molecular weight silicone or other suitable solvent and, thereafter, the solvent can be evaporated. An alternative method comprising hot melt silicones could also be used.

The substrate used in the above processes can be any surface which will impart the desired configuration to the compositions. Thus, it may be a continuous belt onto which the carrier sheet precursor composition or the gel forming composition is spread. Depending on the consistency of the compositions, the substrate may have barriers at its edges to restrict the flow of the compositions until cure takes place.

When it is desired to carry out the manufacture of the adhesive device of this invention as a continuous process, it is generally preferred to preform the carrier sheet as a separate operation. The preformed carrier sheet is then brought into contact with the silicone gel forming composition which is thereafter cured. Thus, for example, the carrier sheet may be laid on the exposed surface of the gel forming composition supported on a suitable substrate, or, alternatively the gel-forming composition may be coated on to the preformed carrier sheet. The gel forming composition is then cured.

In a preferred embodiment of the invention, the substrate is a preformed blister package. As such, the process would comprise (1) depositing the gel forming composition into the blister packaging, (2) applying the 9 carrier sheet on the gel forming composition, (3) applying the second gel forming composition on the exposed surface of the carrier sheet and (4) curing both gel forming compositions and (5) sealing the blister pack.

In this preferred embodiment, the blister pack can be any of the conventional blister packaging materials including, for example, polyvinyl chloride, polypropylene, polyethylene, polyester, paper or composites with or without suitable release coatings. The blister pack could be sealed with conventional materials including, for example, the release liners described above or foils.

The adhesive device used in the present invention can be any size and shape desired based on the final use. For instance, it can be circular, square or rectangular and it can vary from a few square centimeters to in excess of several hundred square cm.

The adhesive devices of this invention are adapted for adhering medical prosthesis on patients. Examples of such prosthesis include devices such as breast prosthesis, catheters, cannulas, drainage bags, uridomes, incontinence devices, pouches, false hairpieces (e.g., toupees), tubes, ostomy and related devices, and the like. In addition, however, the adhesive devices can be used to adhere items such as surgery drapes, facial masks, gloves, and the like.

Accordingly, in another of its aspects the present invention provides a substrate having an adhesive device for adhering it to a second substrate comprising:
a substrate having a surface to be adhered to a second substrate; and
on the surface of the substrate to be adhered to the second substrate, an adhesive device comprising:
   a carrier sheet, said carrier sheet having at least two surfaces;
   on one surface of the carrier sheet is a first, continuous layer of a silicone gel having a density in the range of about 100 to 4500 g/m²; said gel having sufficient tack to adhere to the substrate; and
   on a second surface of the carrier sheet is a second continuous layer of a silicone gel having a density in the range of about 100 to 4500 g/m², said gel having sufficient tack to adhere to the second substrate,
   wherein the first continuous layer of silicone gel of the adhesive device is adhered to the surface of the substrate to be adhered to a second substrate, and wherein the substrate is a prosthesis and the second substrate is the skin of a human or animal body.

In yet another of its aspects the present invention provides a method for adhering a prosthesis to the skin of a human or an animal body comprising:
positioning an adhesive device between the prosthesis and the skin of the human or animal body; and
compressing the adhesive device between the prosthesis and the skin of the human or animal body, wherein the adhesive device comprises:
   a carrier sheet, said carrier sheet having at least two surfaces;
   on one surface of the carrier sheet is a first, continuous layer of a silicone gel having a density in the range of about 100 to 4500 g/m²; said gel having sufficient tack to adhere to the prosthesis; and
   on a second surface of the carrier sheet is a second continuous layer of a silicone gel having a density in the range of about 100 to 4500 g/m², said gel having sufficient tack to adhere to the skin of the human or animal body.

In yet another of its aspects the present invention provides the use of an adhesive device for adhering a first substrate to a second substrate, wherein the adhesive device comprises:
a carrier sheet, said carrier sheet having at least two surfaces;
on one surface of the carrier sheet is a first, continuous layer of a silicone gel having a density in the range of about 100 to 4500 g/m²; said gel having sufficient tack to adhere to the first substrate; and
on a second surface of the carrier sheet is a second continuous layer of a silicone gel having a density in the range of about 100 to 4500 g/m², said gel having sufficient tack to adhere to the second substrate, wherein the first substrate is a prosthesis and the second substrate is the skin of a human or and animal body

The following Examples illustrate the invention. Unless otherwise indicated, all percentages are by weight and all viscosities are at 25°C.

### Example 1

An adhesive device of the present invention was made by dipping a strip of non-woven polypropylene into a gel forming composition. The gel forming composition was made by mixing a vinyl terminated polydimethylsiloxane, a poly(dimethyl,methylhydrogen)siloxane; a hydrogen terminated polydimethylsiloxane; and a platinum catalyst. The gel forming composition was then cured for 30 minutes at room temperature and 30 minutes at 100°C. The resultant adhesive device had 850 g/m² gel on each surface of the polypropylene.

75 cm² of the above adhesive device was used to adhere a 1 kg external breast prosthesis on a vertical plastic surface. The prosthesis was maintained for 48 hours.

### Example 2

Four adhesive devices of the invention were made by the method of Example 1, except that the gel forming composition and its method of application to the polypropylene differed as follows:
Sample 1 - A strip of non-woven polypropylene was dipped into a gel forming composition. The gel forming composition was made by mixing a 50:50 mixture of Part A comprising 99.3% vinyl terminated polydimethylsiloxane and 0.7% platinum complex catalyst and Part B comprising 2% poly(dimethyl,methylhydrogen) siloxane and 98% vinyl terminated polydimethylsiloxane. The gel forming composition was then cured for 15 minutes at room temperature and 30 minutes at 100°C. The resultant adhesive device was again dipped in the same gel forming composition and then cured for 15 minutes at room temperature and 30 minutes at 100°C.
Sample 2 - A strip of non-woven polypropylene was dipped into a gel forming composition. The gel forming composition was made by mixing a 50:50 mixture of Part A comprising 99.8% vinyl-terminated polydimethylsiloxane and 0.2% platinum complex catalyst and Part B comprising 0.1% poly(dimethyl,methylhydrogen) siloxane; 23.3% hydrogen-terminated polydimethylsiloxane; and 76.6% vinyl-terminated polydimethylsiloxane. The gel forming composition was then cured for 120 minutes at room temperature. The resultant adhesive device was again dipped into the same gel forming composition and then cured for 120 minutes at room temperature and 15 minutes at 100°C. The device was post cured for 60 minutes at 100°C.
Sample 3 - A strip of non-woven polypropylene was dipped into a gel forming composition. The gel forming composition was made by mixing a 50:50 mixture of Part A comprising 99.8% vinyl-terminated polydimethylsiloxane and 0.2% platinum complex catalyst and Part B comprising 0.1% poly(dimethyl,methylhydrogen) siloxane; 23.3% hydrogen-terminated polydimethylsiloxane; and 76.6% vinyl-terminated polydimethylsiloxane. The gel forming composition was then cured for 120 minutes at room temperature. The resultant adhesive device was again dipped into the same gel forming composition and then cured for 120 minutes at room temperature and 15 minutes at 100°C.
Sample 4 - A strip of non-woven polypropylene was coated with a gel forming composition by using a bar coater. The gel forming composition was made by mixing 83% vinyl-terminated polydimethylsiloxane, 0.1% platinum complex catalyst, 4% poly(dimethyl,methylhydrogen) siloxane and 13% vinyl functional polydimethylsiloxane. The gel forming composition was then cured for 90 minutes at 100°C.

The adhesive strength of these materials was measured by a peel test. This test involved a 180 degree peel of the samples from a rigid silicone rubber panel. A tensile machine was used to measure the amount of force necessary to peel the samples. The results are presented in Table 1.

**Table 1**

| | |
|---|---|
| Sample | Mean Force (N/cm²) |
| 1 | 0.17 |
| 2 | 0.42 |
| 3 | 0.81 |
| 4 | 3.85 |

This Example demonstrates the wide variation in tack of silicone gels.

### Example 3

Five 100 g samples of different gel forming compositions were prepared, deposited in paper cups and cured.

Sample 1 - The gel forming composition was made by mixing vinyl-terminated polydimethylsiloxane, platinum complex catalyst, and poly(dimethyl,methylhydrogen) siloxane.

Sample 2 - The gel forming composition was made by mixing 52.3% vinyl-terminated polydimethylsiloxane, 0.04% platinum complex catalyst, 2.5% poly(dimethyl, methylhexenyl)siloxane, 16.1% of trimethoxysilylethyl terminated polydimethylsiloxane, 0.2% poly(dimethyl, methylhydrogen)siloxane, 2.5% hydrogen-terminated polydimethylsiloxane and 26.4% 20cSt polydimethylsiloxane.

Sample 3 - The gel forming composition was made by mixing 55.1% vinyl-terminated polydimethylsiloxane, 0.04% platinum complex catalyst, 7.7% of trimethoxysilylethyl terminated polydimethylsiloxane, 0.3% poly(dimethyl, methylhydrogen) siloxane, 3.2% hydrogen-terminated polydimethylsiloxane and 33.6% 20cSt polydimethylsiloxane.

Sample 4 - The gel forming composition was made by mixing 44% vinyl-terminated polydimethylsiloxane, 0.04% platinum complex catalyst, 4.4% poly(dimethyl, methylhexenyl)siloxane, 22.9% of trimethoxysilylethyl terminated polydimethylsiloxane, 0.1% poly(dimethyl, methylhydrogen) siloxane, 1.7% hydrogen-terminated polydimethylsiloxane and 26.9% 20cSt polydimethylsiloxane.

Sample 5 - The gel forming composition was made by mixing 48.2% vinyl-terminated polydimethylsiloxane, 0.04% platinum complex catalyst, 4.8% poly(dimethyl, methylhexenyl)siloxane, 25.1% of trimethoxysilylethylterminated polydimethylsiloxane, 0.3% poly(dimethyl, methylhydrogen) siloxane, 1.8% hydrogen-terminated polydimethylsiloxane and 19.8% 20cSt polydimethylsiloxane.

The gel forming compositions were then cured for 60 minutes at 100°C.

The softness and elasticity of these materials was measured by a penetration-relaxation test with a texture analyzer. The softness of materials is obtained by measuring the penetration in mm of a probe driven into the material with a fixed force. The softness is characteristic of adhesiveness and flexibility of final adhesive devices. The elasticity is obtained by recording the reaction of material subjected a penetration test. The elasticity is characteristic of cohesiveness of the final adhesive device and its ability to be removed and repositioned without damage and leaving residue. The results are presented in Table 2.

**Table 2**

| Sample | Penetration (mm) | % Elasticity |
|---|---|---|
| 1 | 200 | 70 |
| 2 | 136 | 52 |
| 3 | 138 | 74 |
| 4 | 205 | 16 |
| 5 | 88 | 71 |

This Examples demonstrates the wide variation in properties of the silicone gels.

## Claims

1. A method for adhering a first substrate to a second substrate with an adhesive device comprising the use of an adhesive device comprising:
a carrier sheet, said carrier sheet having at least two surfaces;
on one surface of the carrier sheet is a first, continuous layer of a silicone gel having a density in the range of about 100 to 4500 g/m²; said gel having sufficient tack to adhere to the first substrate; and
on a second surface of the carrier sheet is a second continuous layer of a silicone gel having a density in the range of about 100 to 4500 g/m², said gel having sufficient tack to adhere to the second substrate
wherein the first substrate is a prosthesis and the second substrate is the skin of a human or an animal body.

2. The method according to Claim 1 in which the carrier sheet is non-woven and continuous and is made from a material selected from the group consisting of polysaccharide based materials, polyethylene, polyamide, polyurethane, nylon, polyester, polypropylene, polytetrafluoroethylene, and silicone.

3. The method according to any of the previous Claims in which the carrier sheet has a density of about 5 to 150 g/m² and a thickness in the range of about 0.01 to about 1 mm.

4. The method according to any of the previous Claims in which the first and second continuous layers of silicone gel are formed by the reaction of a silicone having Si-H groups with a silicone having Si-aliphatically unsaturated groups in the presence of a platinum or rhodium catalyst.

5. The method according to any of the previous Claims in which the first and second continuous layers of silicone gel have a thickness in the range of about 0.2 to 5 mm.

6. The method according to any of the previous Claims in which the first and second continuous layers of silicone gel are covered by release liners.

7. A substrate having an adhesive device for adhering it to a second substrate comprising:
a substrate having a surface to be adhered to a second substrate; and on the surface of the substrate to be adhered to the second substrate, an adhesive device comprising:
a carrier sheet, said carrier sheet having at least two surfaces;
on one surface of the carrier sheet is a first, continuous layer of a silicone gel having a density in the range of about 100 to 4500 g/m²; said gel having sufficient tack to adhere to the substrate; and
on a second surface of the carrier sheet is a second continuous layer of a silicone gel having a density in the range of about 100 to 4500 g/m², said gel having sufficient tack to adhere to the second substrate,
wherein the first continuous layer of silicone gel of the adhesive device is adhered to the surface of the substrate to be adhered to a second substrate,
and wherein the substrate is a prosthesis and the second substrate is the skin of a human or animal body.

8. A method for adhering a prosthesis to the skin of a human or an animal body comprising:
positioning an adhesive device between the prosthesis and the skin of the human or animal body; and
compressing the adhesive device between the prosthesis and the skin of the human or animal body, wherein the adhesive device comprises:
a carrier sheet, said carrier sheet having at least two surfaces;
on one surface of the carrier sheet is a first, continuous layer of a silicone gel having a density in the range of about 100 to 4500 g/m²; said gel having sufficient tack to adhere to the prosthesis; and
on a second surface of the carrier sheet is a second continuous layer of a silicone gel having a density in the range of about 100 to 4500 g/m², said gel having sufficient tack to adhere to the skin of the human or animal body.

9. The use of an adhesive device for adhering a first substrate to a second substrate, wherein the adhesive device comprises:
a carrier sheet, said carrier sheet having at least two surfaces;
on one surface of the carrier sheet is a first, continuous layer of a silicone gel having a density in the range of about 100 to 4500 g/m²; said gel having sufficient tack to adhere to the first substrate; and
on a second surface of the carrier sheet is a second continuous layer of a silicone gel having a density in the range of about 100 to 4500 g/m², said gel having sufficient tack to adhere to the second substrate
wherein the first substrate is a prosthesis and the second substrate is the skin of a human or an animal body.

## Patentansprüche

1. Verfahren zum Ankleben eines ersten Substrats an ein zweites Substrat mit einem Klebeelement, das die Verwendung eines Klebeelements umfassend
ein Trägerblatt, wobei dieses Trägerblatt mindestens zwei Oberflächen aufweist,
auf einer Oberfläche des Trägerblatts eine erste kontinuierliche Schicht eines Silicongels mit einer Dichte im Bereich von etwa 100 bis 4500 g/m², wobei dieses Gel ausreichende Klebrigkeit aufweist, um an dem ersten Substrat anzuhaften, und
auf einer zweiten Oberfläche des Trägerblatts eine zweite kontinuierliche Schicht eines Silicongels mit einer Dichte im Bereich von etwa 100 bis 4500 g/m², wobei dieses Gel ausreichende Klebrigkeit aufweist, um an dem zweiten Substrat anzuhaften,
umfasst,
wobei das erste Substrat eine Prothese und das zweite Substrat die Haut eines menschlichen oder tierischen Körpers ist.

2. Verfahren gemäß Anspruch 1, in welchem das Trägerblatt nicht gewebt ist und kontinuierliche ist und aus einem Material hergestellt ist, das ausgewählt ist aus der Gruppe bestehend aus Materialien auf Polysaccharidbasis, Polyethylen, Polyamid, Polyurethan, Nylon, Polyester, Polypropylen, Polytetrafluorethylen und Silicon.

3. Verfahren gemäß einem der vorhergehenden Ansprüche, in welchem das Trägerblatt eine Dichte von etwa 5 bis 150 g/m² und eine Dicke im Bereich von etwa 0,01 bis etwa 1 mm aufweist.

4. Verfahren gemäß einem der vorhergehenden Ansprüche, in welchem die erste und zweite kontinuierliche Schicht aus Silicongel durch die Reaktion eines Silicons, das Si-H-Gruppen aufweist, mit einem Silicon, das Sialiphatisch ungesättige Gruppen aufweist, in Gegenwart eines Platin- oder Rhodiumkatalysators gebildet werden.

5. Verfahren gemäß einem der vorhergehenden Ansprüche, in welchem die erste und zweite kontinuierliche Schicht aus Silicongel eine Dicke im Bereich von etwa 0,2 bis 5 mm aufweisen.

6. Verfahren gemäß einem der vorhergehenden Ansprüche, in welchem die erste und zweite kontinuierliche Schicht aus Silicongel von Trennblättern bedeckt sind.

7. Substrat mit einem Klebeelement zum Ankleben an ein zweites Substrat, umfassend:
ein Substrat mit einer Oberfläche zum Ankleben an ein zweites Substrat und auf der Oberflächen des Substrats, das mit dem zweiten Substrat zu verkleben ist, ein Klebeelement, das umfasst:
ein Trägerblatt, wobei dieses Trägerblatt mindestens zwei Oberflächen aufweist,
auf einer Oberfläche des Trägerblatts eine erste kontinuierliche Schicht eines Silicongels mit einer Dichte im Bereich von etwa 100 bis 4500 g/m²,
wobei dieses Gel ausreichende Klebrigkeit aufweist, um an dem ersten Substrat anzuhaften, und
auf einer zweiten Oberfläche des Trägerblatts eine zweite kontinuierliche Schicht eines Silicongels mit einer Dichte im Bereich von etwa 100 bis 4500 g/m², wobei dieses Gel ausreichende Klebrigkeit aufweist, um an dem zweiten Substrat anzuhaften,
wobei die erste kontinuierliche Schicht aus Silicongel des Klebeelements mit der Oberfläche des Substrats, das an das zweite Substrat angeklebt werden soll, verklebt ist und
wobei das erste Substrat eine Prothese und das zweite Substrat die Haut eines menschlichen oder tierischen Körpers ist.

8. Verfahren zum Verkleben einer Prothese mit der Haut eines menschlichen oder tierischen Körpers, umfassend:
Anordnen eines Klebeelements zwischen der Prothese und der Haut des menschlichen oder tierischen Körpers und
Zusammenpressen des Klebeelements zwischen der Prothese und der Haut des menschlichen oder tierischen Körpers, wobei das Klebeelement umfasst:
ein Trägerblatt, wobei dieses Trägerblatt mindestens zwei Oberflächen aufweist,
auf einer Oberfläche des Trägerblatts eine erste kontinuierliche Schicht eines Silicongels mit einer Dichte im Bereich von etwa 100 bis 4500 g/m²,
wobei dieses Gel ausreichende Klebrigkeit aufweist, um an der Prothese anzuhaften, und
auf einer zweiten Oberfläche des Trägerblatts eine zweite kontinuierliche Schicht eines Silicongels mit einer Dichte im Bereich von etwa 100 bis 4500 g/m², wobei dieses Gel ausreichende Klebrigkeit aufweist, um an der Haut des menschlichen oder tierischen Körpers anzuhaften.

9. Verwendung eines Klebeelements zum Verkleben eines ersten Substrats mit einem zweiten Substrat, wobei das Klebeelement umfasst:
ein Trägerblatt, wobei dieses Trägerblatt mindestens zwei Oberflächen aufweist,;
auf einer Oberfläche des Trägerblatts eine erste kontinuierliche Schicht eines Silicongels mit einer Dichte im Bereich von etwa 100 bis 4500 g/m²,
wobei dieses Gel ausreichende Klebrigkeit aufweiset, um an dem ersten Substrat anzuhaften, und
auf einer zweiten Oberfläche des Trägerblatts eine zweite kontinuierliche Schicht eines Silicongels mit einer Dichte im Bereich von etwa 100 bis 4500 g/m², wobei dieses Gel ausreichende Klebrigkeit aufweist, um an dem zweiten Substrat anzuhaften,
wobei das erste Substrat eine Prothese und das zweite Substrat die Haut eines menschlichen oder tierischen Körpers ist.

## Revendications

1. Procédé pour coller un premier substrat à un deuxième substrat avec un dispositif adhésif comprenant l'utilisation d'un dispositif adhésif comprenant :
une feuille de support, ladite feuille de support ayant au moins deux surfaces ;
sur une surface de la feuille de support est placé une première couche continue d'un gel de silicone ayant une masse volumique dans la plage d'environ 100 à 4500 g/m²; ledit gel ayant une adhésivité suffisante pour adhérer au premier substrat ; et
sur une deuxième surface de la feuille de support est située une deuxième couche continue d'un gel de silicone ayant une masse volumique dans la plage d'environ 100 à 4500 g/m² ; ledit gel ayant une adhésivité suffisante pour adhérer au deuxième substrat,
où le premier substrat est une prothèse et le deuxième substrat est la peau d'un corps humain ou animal.

2. Procédé selon la revendication 1 dans lequel la feuille de support est non-tissée et continue et est constituée d'un matériau choisi dans le groupe constitué de matériaux à base de polysaccharide, polyéthylène, polyamide, polyuréthane, nylon, polyester, polypropylène, polytétrafluoroéthylène, et silicone.

3. Procédé selon l'une quelconque des revendications précédentes dans lequel la feuille de support a une masse volumique d'environ 5 à 150 g/m² et une épaisseur dans la plage d'environ 0,01 à environ 1 mm.

4. Procédé selon l'une quelconque des revendications précédentes dans lequel les première et deuxième couches continues de gel de silicone sont formées par la réaction d'une silicone ayant des groupes Si-H avec une silicone ayant des groupes à instauration Si-aliphatique en présence d'un catalyseur au platine ou au rhodium.

5. Procédé selon l'une quelconque des revendications précédentes dans lequel les première et deuxième couches continues de gel de silicone ont une épaisseur dans la plage d'environ 0,2 à 5 mm.

6. Procédé selon l'une quelconque des revendications précédentes dans lequel les première et deuxième couches continues de gel de silicone sont recouvertes par des revêtements antiadhésifs.

7. Substrat ayant un dispositif adhésif pour coller celui-ci à un deuxième substrat comprenant :
un substrat ayant une surface à coller à un deuxième substrat ; et sur la surface du substrat à coller au deuxième substrat, un dispositif adhésif comprenant :
une feuille de support, ladite feuille de support ayant au moins deux surfaces ;
sur une surface de la feuille de support est placé une première couche continue d'un gel de silicone ayant une masse volumique dans la plage d'environ 100 à 4500 g/m² ; ledit gel ayant une adhésivité suffisante pour adhérer au premier substrat ; et
sur une deuxième surface de la feuille de support est situé une deuxième couche continue d'un gel de silicone ayant une masse volumique dans la plage d'environ 100 à 4500 g/m² ; ledit gel ayant une adhésivité suffisante pour adhérer au deuxième substrat,
où la première couche continue de gel de silicone du dispositif adhésif est collée à la surface du substrat à coller à un deuxième substrat,
et où le premier substrat est une prothèse et le deuxième substrat est la peau d'un corps humain ou animal.

8. Procédé pour coller une prothèse à la peau d'un corps humain ou animal comprenant :
le positionnement d'un dispositif adhésif entre la prothèse et la peau du corps humain ou animal ; et
la compression du dispositif adhésif entre la prothèse et la peau du corps humain ou animal, où le dispositif adhésif comprend :
une feuille de support, ladite feuille de support ayant au moins deux surfaces ;
sur une surface de la feuille de support est placé une première couche continue d'un gel de silicone ayant une masse volumique dans la plage d'environ 100 à 4500 g/m²; ledit gel ayant une adhésivité suffisante pour adhérer à la prothèse ; et
sur une deuxième surface de la feuille de support est située une deuxième couche continue d'un gel de silicone ayant une masse volumique dans la plage d'environ 100 à 4500 g/m² ; ledit gel ayant une adhésivité suffisante pour adhérer à la peau du corps humain ou animal.

9. Utilisation d'un dispositif adhésif pour coller un premier substrat à un deuxième substrat, où le dispositif adhésif comprend :
une feuille de support, ladite feuille de support ayant au moins deux surfaces ;
sur une surface de la feuille de support est placé une première couche continue d'un gel de silicone ayant une masse volumique dans la plage d'environ 100 à 4500 g/m²; ledit gel ayant une adhésivité suffisante pour adhérer au premier substrat ; et
sur une deuxième surface de la feuille de support est situé une deuxième couche continue d'un gel de silicone ayant une masse volumique dans la plage d'environ 100 à 4500 g/m² ; ledit gel ayant une adhésivité suffisante pour adhérer au deuxième substrat,
où le premier substrat est une prothèse et le deuxième substrat est la peau d'un corps humain ou animal.
